# EUROPEAN PATENT SPECIFICATION

(11) **EP 2 004 092 B1**
(45) Date of publication and mention of the grant of the patent: **29.07.2015**
(21) Application number: 07753839.5
(22) Date of filing: 23.03.2007
(51) Int. Cl.: A61B 17/70

(54) **SPACING MEANS FOR INSERTION BETWEEN SPINOUS PROCESSES OF ADJACENT VERTEBRAE**
ZWISCHENELEMENTE FÜR DEN EINSATZ ZWISCHEN DORNFORTSÄTZEN ANGRENZENDER WIRBEL
MOYENS D'ESPACEMENT POUR INSERTION ENTRE DES APOPHYSES ÉPINEUSES DE VERTÈBRES ADJACENTES

(30) Priority: 24.03.2006 GB 0605962
(43) Date of publication of application: 24.12.2008
(73) Proprietor: EBI, LLC, Parsippany, NJ 07054 (US)
(72) Inventor: GALLEY, Geoffrey Harrison, Totteridge, London N20 8PJ (GB); ALLIBONE, James Bernard, Radlett, Herts WD7 7DQ (GB); NOORDEEN, Mohammed Hamza Hilali, London W14 8JH (GB); TAYLOR, Benjamin Anthony, Tring, Herts HP23 5QN (GB); TUCKER, Stewart Kenneth, London N6 4RT (GB)
(74) Representative: Gross, Felix
(86) International application number: PCT/US2007/007243
(87) International publication number: WO 2007/111999

(56) References cited:
- EP-A1- 1 627 616
- WO-A1-01/91657
- FR-A1- 2 703 239
- US-A- 4 713 003
- US-A1- 2005 165 398
- US-B1- 6 179 873
- US-B1- 6 494 883

## Description

We, Geoffrey Harrison Galley, of Red Lodge, The Close, Totteridge London N20 8PJ, James Bernard Allibone of 17 The Avenue, Radlett, Herts WD7 7DQ, Mohammed Hamza Hilali Noordeen of 42 Addison Rd, London W14 83H, Benjamin Anthony Taylor of Marshcroft Farm, Marshcroft Lane, Tring, Herts HP23 5QN and Stewart Kenneth Tucker of West Cottage, 21 Hampstead Lane, London N6 4RT, British subjects, do hereby declare this invention, which is described in and by the following statement.

This invention relates to the insertion of one or more spacing means in the human vertebral column and is an improved means of provision and insertion off such spacing means. Recent advances in minimally invasive spinal surgery have led to the adoption of spacing means in order to increase the distance between adjacent spinous processes extending from the rear of the spinal vertebrae. Such spacing means are presently marketed by U. S. companies such as Medtronics Inc. and Saint Francis Medical Inc.

Further spacing means or tools are also known. US 6,916,321 B2 discloses an orthopedic surgical interference screw including a front component and a rear component. A tool may be used to insert the respective front components and rear components into a prepared portion of a bone. US 5,209,753_discloses a bone screw having a threaded portion and a head. A shaft is screwed into a longitudinal bore of the bone screw and is subsequently attached to a tip via a bore formed in the tip. A longitudinal bore extends through the interior of the screw. A tapered conical portion is provided at the free end opposite to the head. US 2005 / 165 398 A1 discloses a device that is suitable for non-invasive insertion of an enlargeable or expandable structure comprising a hinge, a top arm and a bottom arm, wherein the arms and define an angle of taper. The desired angle of taper is created by fixation members, such as screws and fixation member receivers, such as holes or bores; which allow for controlled expansion and independent right and left side adjustment in order to achieve the desired inclined planes. EP 1 627 616 A1 discloses a spacer and insertion instrument that facilitates insertion of the spacer between adjacent spinous processes. The insertion instrument includes a grasp portion and support portion disposed on opposite ends of the insertion instrument. FR 2 703 239 relates to a device that appears to be used to fix a strap that extends around adjacent spinous processes. WO 01 / 91657 A1 relates to a supplemental spine fixation device which includes a guide and a spacer for distracting apart adjacent spinous processes and the device also comprises hook members which hook about the first and second spinous processes.

The present spacing means each suffer a distinct disadvantage in that due to their size and other considerations neither of them is suitable for use in the cervical spine. Further the said spacing means each require the creation of a significant surgical trauma in order to provide access for the insertion of the said spacing means into the human body. It is an object of the present invention to provide a spacing means which can be positioned between the spinous processes using only a minimally invasive surgical procedure thereby reducing the degree of trauma suffered by the patient and hence the time required for the healing of the insertion wound.

The present invention relates to a device for inserting a spacer between adjacent spinous processes of adjacent vertebrae as claimed hereafter. Preferred embodiments of the invention are set forth in the dependent claims.

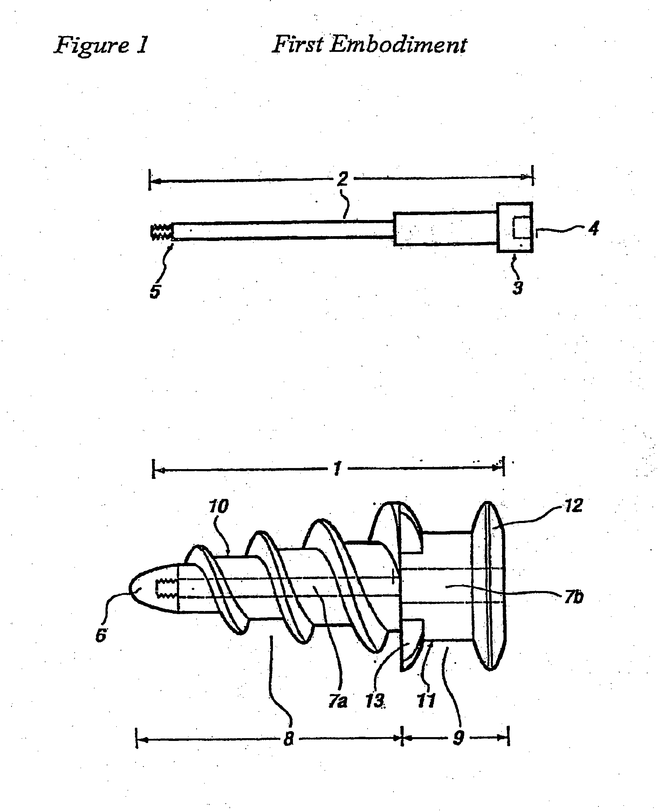

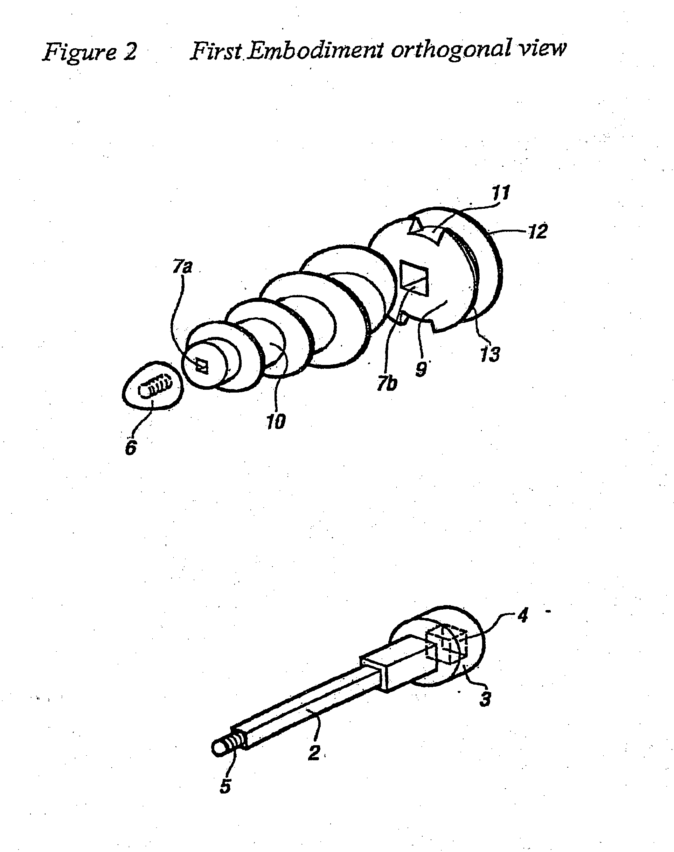

The present invention is described by reference to Figure 1 and Figure 2 which respectively represent a plan view and an orthogonal view of a first embodiment of the device of the invention. The device consists of a spacing means in the form of a segmented screw-like assembly (1) which is held together by means of a shaped rod. (2). One end of said shaped rod (2) is provided with a concentric cap (3) having a recess (4) into which a tool may be inserted in order to rotate the said shaped rod. The other end of the said shaped rod is provided with a screwed portion (5), onto which a threaded shaped "blind" nut (6) may be screwed. The said shaped rod (2) is formed with a square cross-section so as to engage with similarly shaped holes (7a and 7b) provided through the center of the portions of the device. The cross-sectional dimensions of the length of the said shaped rod engaging with the distinct portions (8) & (9) of the device are different so as to provide for the separate rotation of different portions of the device.

The first portion (8) of the said first embodiment of the device is comprised of one or more components, which singly or together form a section of a tapered screw the root of which (10) may be advantageously disposed more or less parallel to the longitudinal axis of the device and which may be of a form (not shown) appropriate for the location of one or more spinous processes which may rest on the said root.

The second portion (9) of the said first embodiment of the device comprises a spacing member which will be inserted between the said processes of the said spinal vertebrae during the required surgical procedure and which will remain in position upon the completion of the surgical procedure. The said second portion comprises a central hub (11) of a form which is adapted so as to provide a surface upon which the said spinous processes may bear when the said second portion rests between two such spinous processes. The cross-sectional profile of the said hub, which lies at right angles to the horizontal axis through the center of the component may vary along the direction of the said horizontal axis so as to provide lateral location for the spinous processes, which rest against it (not shown).

As can be seen in Figures 1 &2 the hub section (11) of the second portion of the said first embodiment of the device is provided at either end with a terminating annulus (12) or part annulus (13). The said part annulus may be orientated (not shown) so as to assist the forward movement of the said second portion of the first embodiment of the device as the assembled device is rotated about its axis in engagement with the said spinous processes and their surrounding tissue..

In order to effect the insertion of the spacing member (9) the surgeon first provides access to the left and right hand sides of the adjacent spinous processes between which it is desired to insert the said spacing member using minimally invasive techniques known to those skilled in the surgical art. The assembled first embodiment of the device is inserted into the area between the adjacent spinous processes and rotated using a driving tool (not shown) which may be advantageously fitted with a ratchet device for ease of operation. As the said first embodiment of the device advances through the space between the adjacent spinous processes the increasing cross-sectional dimensions of the root of the first portion of the device causes the adjacent spinous processes to progressively separate.

The said second component (9) of the first embodiment of the device is selected by the surgeon to provide the desired spacing between the adjacent spinous processes upon completion of the surgical procedure. When the said second component of the device enters the space between the adjacent spinous processes and has been set at the appropriate rotational angle, the leading end of the first portion of the screw-like device may be accessed on the left-hand side of the spinous processes. The shaped nut (6) which has been screwed on to the shaped rod (2) during the assembly of the device may now be gripped using a surgical pliers and rotated so as to remove it from the screwed end of the shaped rod. Alternatively the shaped nut (6) may be provided with a means of attaching a special-purpose tool (not shown) which may in turn be provided with a ratchet for ease of operation in removing the shaped nut from the said shaped rod.

Following the removal of the shaped nut (6) from the shaped rod (2), the shaped rod is withdrawn by the surgeon from the right hand side of the spinous processes. At this point, a second shaped rod (not shown) having cross-sectional dimensions similar to those of the said square section hole (7a) in the said first component of the first embodiment of the device, but smaller than those of the square section hole (7b) in the said second component (9) of the device (alternatively referred to as "the spacing member") is inserted from the right hand side of the spinous processes and fed through the larger square hole (7b) in the said spacing member (9) so as to pass through the smaller square section hole (7a) in the said first component of the first embodiment of the device. The shaped nut (6) is now screwed onto the threaded end of the said second shaped rod and the first component (8) of the device is now rotated by means of the smaller shaped rod so as to drive it forward, releasing it from the the patient's body to the left of the spinous processes.. The said shaped nut (6) is now unscrewed from the said second shaped rod and the rod is withdrawn from the body of the patient on the right hand side of the spinous processes leaving the said second component (or spacing member) (9) of the said segmented tapered screw assembly disposed between the spinous processes of the required adjacent vertebrae. In performing the surgical procedure the insertion of the spacing member may, if desired, be made from the left hand side of the spinous processes in which event the instructions as to left hand and right band positioning are interchanged. The spacing member (9) may, if desired, be provided with an attached clip or suture (not shown) by means of which it may be secured to the adjacent spinous processes in order to prevent dislodgement.

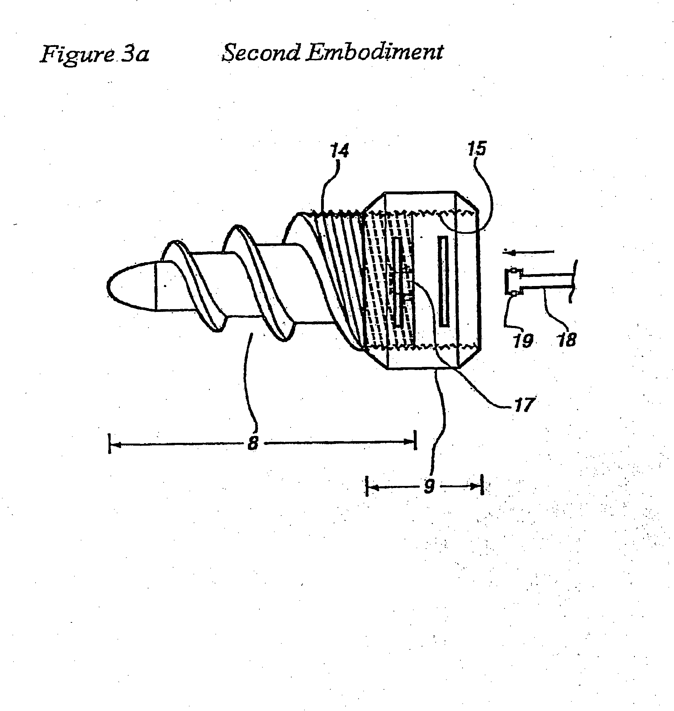

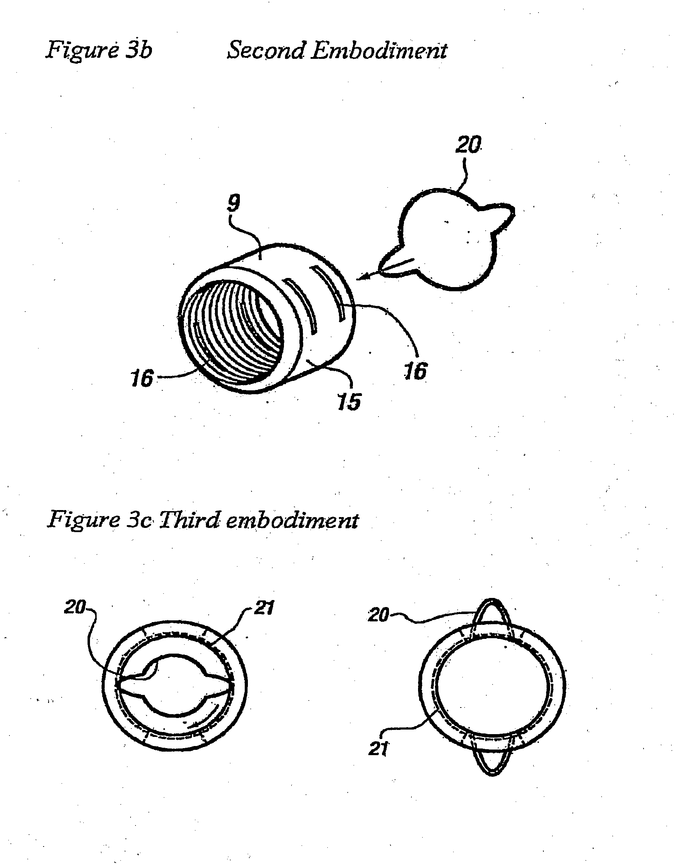

In a second and preferred embodiment of the device shown in Figure 3a, the first component (8) of the device is provided with a threaded portion (14) which engages with with the internally threaded bore (15) of the second component (9) of the device otherwise again referred to as " the spacing member". The said second, component (9) may be advantageously provided with a means (not shown) of limiting the extent to which the first portion may travel along the bore of the second component and project beyond the end of the second component. The second component is further provided with four or more slots (16) through which sprung members may project upon final disposition of the device as shown in figure 3b. at (1).

The assembled device is inserted using a shaped rod the end portion of which is shown at (18 in fig 3a) which may advantageously be of a flexible nature. The said shaped rod locks into a recess (17) in the first component (8) of the second embodiment of the device by means of a sprung ball fitting at its end (19). Following the positioning of the spacing member (9) the first component of the device (8) is withdrawn down the screwed bore (15) of the the said spacing member. Clips (20 in fig 3b) are then entered into the open bore of the second component using a compressing tool (not shown) which releases the clips within the bore to project through the slots provided in the spacing member (9).

In a further embodiment shown in figure 3b, the clips (20) are disposed within the bore of the second component behind the extended first component upon insertion of the assembled device. After positioning of the spacing member between the spinous processes in the manner described above with reference to Figures 1 and 2, the clips are repositioned to project through the slots with the non-projecting portion of the clips seated in a housing groove 21 set in the screwed internal surface of the bore of the spacing members as shown in figure 3c. The first component is then screwed back through the bore of the said spacing member and withdrawn from the body of the patient as described in relation to the second embodiment of the device but in this case after passing over the seated portions of the locating clips.

The device of the invention may be fabricated from any material, which is suitable for use in the human body. The preferred material for first and second components as well as the blind nut may be any plastic material, which has been formulated for use in the human body. The said plastic material may be coated or treated with other material in order to render it more compatible with surrounding tissue. The spring clips referred to in relation to second and third embodiments may be advantageously formed from stainless spring steel or any suitable metal alloy.

In order to facilitate viewing of the device during insertion in the human body, the material of the said flexible enclosure may be rendered radio-opaque by inclusion therein of a radio opaque material in forms familiar to those skilled in the art of radiographic imaging.

The device of the invention may be manufactured in a variety of sizes suitable for insertion at different points in the spinal column. First and second components of different sizes may be combined to achieve the best result in any given location. The device of the invention has the advantage of limiting the necessary independent displacement of the adjacent vertebrae to the required separation, which is achieved automatically upon the positioning of the spacing member of the device between the adjacent spinous processes. It is thus unnecessary to increase the separation of the adjacent vertebrae beyond the desired separation in order to facilitate the insertion of the spacing member.

## Claims

1. A device for inserting a spacer between adjacent spinous processes of adjacent vertebrae comprising
a screw (8) and a cylindrical spacer (9),
the screw (8) having a body that tapers from a first end to a second end,
with at least one thread formed on an exterior surface of the body to enable the screw (8) to pass through an anatomy,
a rod (2) having a threaded end (5),
wherein the rod (2) comprises a first portion for engaging with the spacer (9) and a second portion for engaging with the screw (8) and wherein said first and said second portion of the rod (2) have different cross-sectional dimensions so as to provide for separate rotation of the different portions,
a cap (3) opposite the threaded end, and
an intermediate section separating the threaded end (5) from the cap (3),
wherein the taper of the screw (8) can progressively separate the adjacent spinous processed, wherein
the spacer (9) defines a bore (7b) that receives the first portion of the intermediate section of the rod (2) to removably couple the spacer (9) to the screw (8),
wherein the spacer (9) can support the adjacent spinous processes,
wherein the screw (8) can be uncoupled from the spacer (9) after the spacer (9) is positioned between the adjacent spinous processes by removing the rod (2) from the screw (8) and the spacer (9) such that the spacer (9) can remain in position between the adjacent spinous processes after removal of the screw (8),
wherein the body of the screw (8) defines a bore (7a) that receives the second portion of the intermediate section of the rod (2) to position the screw (8) adjacent to the spacer (9) along a length of the intermediate section of the rod (2), with the screw (8) sized so that the threaded end (5) of the rod (2) extends beyond an end of the screw (8).

2. The device of Claim 1, further comprising:
a nut (6) that threadably engages the threaded end (5) of the rod (2) so that the rod (2) is disposed entirely within the nut (6), body of the screw (8) and the spacer (9); and
wherein the intermediate section of the rod (2) is sized so that the rod (2) passes through the body of the screw (8) upon removal of the nut (6) from the threaded end (5).

3. The device of Claim 1, wherein the cap (3) is concentric with the rod (2) and defines a recess (4) adapted to enable a driver to rotate the rod (2).

4. The device of Claim 1, wherein the intermediate section of the rod (2) has a square cross-section to enable the body of the screw (8) and the spacer (9) to rotate with the rod (2).

5. The device of Claim 1, wherein the spacer (9) further comprises:
a first formed hub (11) that is adapted to support a respective one of the adjacent spinous processes; and
a second formed hub (11) opposite the first formed hub (11) and adapted to support the other of the adjacent spinous processes.

6. The device of Claim 5, wherein the spacer (9) further comprises:
a first annulus (12) extending from the spacer (9);
a second annulus (13) extending from the spacer (9), the second annulus (13) spaced apart from the first annulus (12) and positioned adjacent to the screw (8) when the screw (8) and spacer (9) are coupled to the rod (2); and
wherein the first hub (11) and the second hub (11) are formed through the second annulus.

7. The device of Claim 2, wherein upon removal of the nut (6), the rod (2) and the screw (8) are removable from the anatomy such that the spacer (9) remains disposed between the adjacent spinous processes.

## Patentansprüche

1. Eine Vorrichtung zum Einführen eines Abstandshalters zwischen angrenzenden Dornfortsätzen von angrenzenden Wirbeln umfassend
eine Schraube (8) und einen zylindrischen Abstandshalter (9),
wobei die Schraube (8) einen Körper aufweist, der sich von einem ersten Ende zu einem zweiten Ende hin verjüngt,
mit mindestens einem Gewinde, das auf einer äußeren Oberfläche des Körpers ausgebildet ist, um ein Durchführen der Schraube (8) durch eine Anatomie zu ermöglichen,
eine Stange (2) mit einem Gewindezapfen (5),
wobei die Stange (2) einen ersten Abschnitt zum Eingreifen mit dem Abstandshalter (9) und einen zweiten Bereich zum Eingreifen mit der Schraube (8) umfasst und wobei der erste und der zweite Bereich der Stange (2) unterschiedliche Querschnittsdimensionen aufweisen, um eine separate Rotation der unterschiedlichen Bereiche bereitzustellen,
eine Kappe (3) gegenüberliegend zu dem Gewindezapfen, und
einen Zwischenabschnitt, der den Gewindezapfen (5) von der Kappe (3) trennt, wobei die Verjüngung der Schraube (8) die angrenzenden Dornfortsätze progressiv trennen kann, wobei
der Abstandshalter (9) eine Bohrung (7b) definiert, die den ersten Bereich des Zwischenabschnitts der Stange (2) aufnimmt, um den Abstandshalter (9) mit der Schraube (8) entfernbar zu koppeln,
wobei der Abstandshalter (9) die angrenzenden Dornfortsätze stützen kann,
wobei die Schraube (8) von dem Abstandshalter (9) abgekoppelt werden kann, nachdem der Abstandshalter (9) zwischen den angrenzenden Dornfortsätzen positioniert ist, durch Entfernen der Stange (2) von der Schraube (8) und dem Abstandshalter (9), sodass der Abstandshalter (9) in der Position zwischen den angrenzenden Dornfortsätzen nach Entfernung der Schraube (8) verbleiben kann,
wobei der Körper der Schraube (8) eine Bohrung (7a) definiert, die den zweiten Bereich des Zwischenabschnittes der Stange (2) aufnimmt, um die Schraube (8) angrenzend zu dem Abstandshalter (9) entlang einer Länge des Zwischenabschnitts der Stange (2) zu positionieren, wobei die Schraube (8) so bemessen ist, dass der Gewindezapfen (5) der Stange (2) sich über das Ende der Schraube (8) erstreckt.

2. Vorrichtung gemäß Anspruch 1 des Weiteren umfassend:
eine Mutter (6), die in den Gewindezapfen (5) der Stange (2) schraubbar eingreift, sodass die Stange (2) vollständig innerhalb der Mutter (6), dem Körper der Schraube (8) und dem Abstandshalter (9) angeordnet ist; und
wobei der Zwischenabschnitt der Stange (2) so bemessen ist, dass die Stange (2) durch den Körper der Schraube (8) nach der Entfernung der Mutter (6) von dem Gewindezapfen (5) reicht.

3. Vorrichtung gemäß Anspruch 1, wobei die Kappe (3) konzentrisch zu der Stange (2) ist und eine Aussparung (4) definiert, die angepasst ist, um einem Führer ein Rotieren der Stange (2) zu ermöglichen.

4. Vorrichtung gemäß Anspruch 1, wobei der Zwischenabschnitt der Stange (2) einen quadratischen Querschnitt aufweist, um den Körper der Schraube (8) und dem Abstandshalter (9) eine Drehung mit der Stange (2) zu ermöglichen.

5. Vorrichtung gemäß Anspruch 1, wobei der Abstandshalter (9) des Weiteren umfasst:
eine erste ausgebildete Nabe (11), die angepasst ist, um den jeweils einen der angrenzenden Dornfortsätze abzustützen; und
eine zweite ausgebildete Nabe (11) gegenüber der ersten ausgebildeten Nabe (11) und angepasst, um den anderen der angrenzenden Dornfortsätze abzustützen.

6. Vorrichtung gemäß Anspruch 5, wobei der Abstandshalter (9) des Weiteren umfasst:
einen ersten Kranz (12), der sich von dem Abstandshalter (9) erstreckt;
einen zweiten Kranz (13) der sich von dem Abstandshalter (9), wobei der zweite Kranz (13) von dem ersten Kranz (12) beabstandet ist und angrenzend zu der Schraube (8) positioniert ist, wenn die Schraube (8) und der Abstandshalter (9) mit der Stange (2) gekoppelt sind; und
wobei die erste Nabe (11) und die zweite Nabe (11) durch den zweiten Kranz ausgebildet werden.

7. Vorrichtung gemäß Anspruch 2, wobei nach Entfernung der Mutter (6) die Stange (2) und die Schraube (8) von der Anatomie entfernbar sind, sodass der Abstandshalter (9) zwischen den angrenzenden Dornfortsätzen angeordnet verbleibt.

## Revendications

1. Dispositif pour insérer un dispositif d'espacement entre des apophyses épineuses adjacentes des vertèbres adjacentes, comprenant :
une vis (8) et un dispositif d'espacement cylindrique (9),
la vis (8) ayant un corps qui se rétrécit progressivement d'une première extrémité à une seconde extrémité,
avec au moins un filetage formé sur une surface extérieure du corps pour permettre à la vis (8) de traverser une anatomie,
une tige (2) ayant une extrémité filetée (5),
dans lequel la tige (2) comprend une première partie pour se mettre en prise avec le dispositif d'espacement (9) et une seconde partie pour se mettre en prise avec la vis (8) et dans lequel ladite première et ladite seconde partie de la tige (2) ont différentes dimensions transversales afin de fournir la rotation séparée des différentes parties,
un capuchon (3) opposé à l'extrémité filetée, et
une section intermédiaire séparant l'extrémité filetée (5) du capuchon (3),
dans lequel la conicité de la vis (8) peut progressivement séparer les apophyses épineuses adjacentes, dans lequel :
le dispositif d'espacement (9) définit un alésage (7b) qui reçoit la première partie de la section intermédiaire de la tige (2) pour coupler, de manière amovible, le dispositif d'espacement (9) à la vis (8),
dans lequel le dispositif d'espacement (9) peut supporter les apophyses épineuses adjacentes,
dans lequel la vis (8) peut être découplée du dispositif d'espacement (9) après que le dispositif d'espacement (9) a été positionné entre les apophyses épineuses adjacentes en retirant la tige (2) de la vis (8) et du dispositif d'espacement (9), de sorte que le dispositif d'espacement (9) peut rester en position entre les apophyses épineuses adjacentes après le retrait de la vis (8),
dans lequel le corps de la vis (8) définit un alésage (7a) qui reçoit la seconde partie de la section intermédiaire de la tige (2) afin de positionner la vis (8) adjacente au dispositif d'espacement (9) le long d'une longueur de la section intermédiaire de la tige (2), avec la vis (8) dimensionnée de sorte que l'extrémité filetée (5) de la tige (2) s'étend au-delà d'une extrémité de la vis (8).

2. Dispositif selon la revendication 1, comprenant en outre :
un écrou (6) qui met en prise par filetage l'extrémité filetée (5) de la tige (2) de sorte que la tige (2) est complètement disposée à l'intérieur de l'écrou (6), du corps de la vis (8) et du dispositif d'espacement (9) ; et
dans lequel la section intermédiaire de la tige (2) est dimensionnée de sorte que la tige (2) passe à travers le corps de la vis (8) suite au retrait de l'écrou (6) de l'extrémité filetée (5).

3. Dispositif selon la revendication 1, dans lequel le capuchon (3) est concentrique avec la tige (2) et définit un évidement (4) adapté pour permettre à un dispositif d'entraînement de faire tourner la tige (2).

4. Dispositif selon la revendication 1, dans lequel la section intermédiaire de la tige (2) a une section transversale carrée pour permettre au corps de la vis (8) et au dispositif d'espacement (9) de tourner avec la tige (2).

5. Dispositif selon la revendication 1, dans lequel le dispositif d'espacement (9) comprend en outre :
un premier moyeu formé (11) qui est adapté pour supporter au moins une apophyse respective des apophyses épineuses adjacentes ; et
un second moyeu formé (11) opposé au premier moyeu formé (11) et adapté pour supporter l'autre des apophyses épineuses adjacentes.

6. Dispositif selon la revendication 5, dans lequel le dispositif d'espacement (9) comprend en outre :
un premier espace annulaire (12) s'étendant à partir du dispositif d'espacement (9) ;
un second espace annulaire (13) s'étendant à partir du dispositif d'espacement (9), le second espace annulaire (13) étant espacé du premier espace annulaire (12) et positionné de manière adjacente à la vis (8) lorsque la vis (8) et le dispositif d'espacement (9) sont couplés à la tige (2) ; et
dans lequel le premier moyeu (11) et le second moyeu (11) sont formés à travers le second espace annulaire.

7. Dispositif selon la revendication 2, dans lequel, suite au retrait de l'écrou (6), la tige (2) et la vis (8) sont amovibles de l'anatomie de sorte que le dispositif d'espacement (9) reste disposé entre les apophyses épineuses adjacentes.
